# EUROPÄISCHE PATENTSCHRIFT

(11) **EP 3 682 972 B1**
(45) Veröffentlichungstag und Bekanntmachung des Hinweises auf die Patenterteilung: **10.04.2024**
(21) Anmeldenummer: 19152388.5
(22) Anmeldetag: 17.01.2019
(51) Int. Cl.: B05B 11/00, G16H 20/10, B05B 11/04, A61M 11/00, A61M 15/00

(54) **SPENDER ZUM AUSTRAG VON FLÜSSIGKEIT, INSBESONDERE ZUM AUSTRAG EINER PHARMAZEUTISCHEN FLÜSSIGKEIT, SOWIE SET UMFASSEND EINEN SOLCHEN SPENDER**
DISPENSER FOR DISCHARGING LIQUID, IN PARTICULAR FOR DISCHARGING A PHARMACEUTICAL LIQUID, AND SET COMPRISING SUCH A DISPENSER
DISTRIBUTEUR DESTINÉ À L'APPLICATION DU LIQUIDE, EN PARTICULIER À L'APPLICATION D'UN LIQUIDE PHARMACEUTIQUE AINSI QU'ENSEMBLE COMPRENANT UN TEL DISTRIBUTEUR

(43) Veröffentlichungstag der Anmeldung: 22.07.2020
(73) Patentinhaber: Aptar Radolfzell GmbH, 78315 Radolfzell (DE)
(72) Erfinder: Keppner, Frank, 72458 Albstadt (DE)
(74) Vertreter: Witte, Weller und Partner Patentanwälte mbB Stuttgart

(56) Entgegenhaltungen:
- EP-A1- 0 511 894
- EP-A1- 3 043 156
- EP-A1- 3 376 182
- EP-A2- 2 708 289
- WO-A1-2011/115484
- WO-A1-2018/165326

## Beschreibung

Die Erfindung betrifft einen Spender zum Austrag von Flüssigkeit, insbesondere zum Austrag einer pharmazeutischen Flüssigkeit.

Ein gattungsgemäßer Spender verfügt ebenso wie ein erfindungsgemäßer Spender über ein Gehäuse mit einer Austragöffnung, durch die hindurch Flüssigkeit aus einem Flüssigkeitsspeicher des Spenders abgegeben werden kann. Dies kann beispielsweise dadurch erfolgen, dass der Flüssigkeitsspeicher als Quetschflasche ausgebildet ist und zum Zwecke des Austrags manuell zusammengedrückt wird.

Durch den Austrag wird bei gattungsgemäßen bzw. erfindungsgemäßen Spendern durch das abgegebene Flüssigkeitsvolumen einen Unterdruck im Flüssigkeitsspeicher erzeugt. Zum Ausgleich dieses Flüssigkeitsvolumen weist der Spender einen Belüftungskanal auf, durch den Luft in den Flüssigkeitsspeicher eingesogen werden kann. Der Belüftungskanal kann zum Zwecke der Vermeidung von Verunreinigungen mit einer Filteranordnung versehen sein.

Je nach Ausgestaltung eines solchen Spenders kann dieser zum Austrag einer baulich definierten Flüssigkeitsdosis je Betätigung oder eine durch die Art der Betätigung durch den Benutzer frei bestimmbaren Flüssigkeitsmenge ausgebildet sein.

Bei einem Spender gattungsgemäßer oder erfindungsgemäßer Art kann es wünschenswert sein, die ausgetragene Flüssigkeitsmenge oder die Anzahl der ausgetragenen Flüssigkeitsdosen zu erfassen, insbesondere durch elektronische Messmittel. Dies kann sowohl im Regelbetrieb eines solchen Spenders beim Endkunden als auch in einer Erprobungsphase durch besondere Produkttester gewollt sein. Zweck kann beispielsweise sein, die Restmenge von Flüssigkeit im Flüssigkeitsspeicher abzuschätzen oder eine Erinnerung des Nutzers zu ermöglichen, wenn ein vorgesehener Austrag nicht stattgefunden hat.

Aus dem Stand der Technik sind bereits vielfältige Messsysteme für Flüssigkeitsspender bekannt, bei denen die Betätigung selbst erfasst und insbesondere in Form einer Zählung verarbeitet wird. Es ist bei solchen Systemem jedoch nicht in jedem Fall gewährleistet, dass tatsächlich ein korrekter Austrag stattgefunden hat, beispielsweise dann nicht, wenn der Spender bei der Betätigung nicht bestimmungsgemäß ausgerichtet war.

Ebenfalls wurde bereits erwogen, den Flüssigkeitsstrom selbst zu erfassen, also eine hierfür geeignete Messeinrichtung in einem Austragkanal zwischen dem Flüssigkeitsspeicher und der Austragöffnung zu sensieren. Dies ist jedoch baulich häufig schwierig zu realisieren und führt zu einer wesentlichen Änderung der Konstruktion eines Spenders. Auch ist je nach verwendeten Materialien einer entsprechenden Sensorik der Flüssigkeitskontakt mit dieser Sensorik nachteilig für die Sensorik und/oder die Flüssigkeit.

Aus der EP 3 376 182 A1 ist ein Fluidaustragsystem bekannt, welches über eine Messeinheit verfügt, durch die hindurch einem Flüssigkeitsspeicher Gas zugeführt werden kann. Die Messeinheit ermittelt dabei die Gasmenge und gestattet au Basis dessen einen Rückschluss auf eine zuvor ausgetragene Flüssigkeitsmenge.

Auch das Dokument EP 3 043 156 A1 beschreibt, dass Messungen im Kontext eines Zuflusses von Gas in einen Flüssigkeitsspeicher genutzt werden, um die auszutragende Flüssigkeitsmenge zu steuern oder die ausgetragene Flüssigkeitsmenge zu ermitteln. Dokument EP 0 511 894 A1 beschreibt einen Spender gemäß dem Oberbegriff von Anspruch 1.

### AUFGABE UND LÖSUNG

Aufgabe der Erfindung ist es, einen Spender zur Verfügung zu stellen, der in vorteilhafter Art und Weise die Erfassung des Flüssigkeitsaustrags gestattet und dabei die oben genannten Probleme des Standes der Technik vermeidet oder vermindert.

Erfindungsgemäß wird hierfür ein Spender zum Austrag von Flüssigkeit, insbesondere zum Austrag einer pharmazeutischen Flüssigkeit, vorgeschlagen.

In Übereinstimmung mit gattungsgemäßen Spendern weisen solche erfindungsgemäßen Spender ein Gehäuse mit einer Austragöffnung zum Austrag von Flüssigkeit sowie einen Flüssigkeitsspeicher zur Lagerung der Flüssigkeit vor dem Austrag auf. Es handelt sich bei den erfindungsgemäßen Spendern um tragbare Spender für den Endkunden und insbesondere für Patienten, gemeinsam im Weiteren Benutzer genannt. Das Maximalvolumen des Flüssigkeitsspeichers beträgt daher üblicherweise zwischen 5 ml und 500 ml, insbesondere zwischen 5 ml und 50 ml.

Die Flüssigkeit im Flüssigkeitsspeicher wird bei Betätigung durch den Benutzer durch einen Austragkanal zur Austragöffnung gefördert. Der Austragöffnung ist vorzugsweise ein Auslassventil vorgeschaltet, welches erst bei einem definierten Grenzdruck öffnet. Die Austragsform kann insbesondere die eines Flüssigkeitsstroms, eines mit Luft vermengten zerstäubten Sprühstrahls oder die Form von Einzeltropfen sein. Im Falle eines Tropfenspenders ist die Austragöffnung von einer Tropfenbildungsfläche umgeben, an der die abzugebende Flüssigkeit anhaftet, bis die Flüssigkeitsmenge ausreichend groß ist, um sich in Tropfenform von der Tropfenbildungsfläche zu lösen. Die Tropfenbildungsfläche ist vorzugsweise außenseitig von einer Abrisskante begrenzt, um eine besonders einheitliche Tropfengröße zu gewährleisten.

Für die Förderung der Flüssigkeit vom Flüssigkeitsspeicher zur Austragöffnung kann insbesondere eine manuell betätigbare Pumpeinrichtung mit Pumpkammer sowie eingangsseitigem und ausgangsseitigem Pumpventilen vorgesehen sein. Eine besonders wesentliche Alternative hierzu ist die Gestaltung des Spenders als Quetschflaschenspender. In diesem Falle ist der Flüssigkeitsspeicher oder ein umgebender Betätigungskörper elastisch zusammendrückbar, so dass ein Überdruck in der Flüssigkeit erzeugt wird, der eine Förderung in Richtung der Austragöffnung bewirkt.

Bei einem erfindungsgemäßen Spender ist vorgesehen, dass nach erfolgtem Austrag und bei Rückstellung des Flüssigkeitsspeichers oder des umgebenden Betätigungskörpers Luft aus der Umgebung in den Flüssigkeitsspeicher eingesogen wird, um das zuvor ausgetragene Flüssigkeitsvolumen auszugleichen und den Druck im Flüssigkeitsspeicher wieder an den Umgebungsdruck anzupassen.

Ein erfindungsgemäßer Spender verfügt zu diesem Zweck über einen Belüftungskanal, mittels dessen eine umgebende Atmosphäre mit dem Flüssigkeitsspeicher verbunden ist, um nach dem Austrag von Flüssigkeit das Einströmen der Luft aus der Atmosphäre zu gestatten.

Gemäß einem Unteraspekt der Erfindung verfügt der Spender weiterhin über eine Erfassungseinrichtung zur Erfassung eines Austragvorgangs, wobei die Erfassungseinrichtung zur Erfassung der durch den Belüftungskanal einströmenden Luft ausgebildet ist.

Es wird demnach vorgeschlagen, weder die Betätigung des Spenders als solchen noch eine unmittelbare Erfassung des ausgetragenen Flüssigkeitsvolumens für die Erfassung des Austrags heranzuziehen, sondern stattdessen den auf den Austrag folgenden Belüftungsvorgang, im Rahmen dessen Luft, insbesondere aus einer umgebenden Atmosphäre, in den Flüssigkeitsspeicher oder einen umgebenden Betätigungskörper einströmt.

Es hat sich gezeigt, dass hiermit ein Austrag sicher identifiziert werden kann, wobei je nach Ausgestaltung nur das Stattfinden des Austrags als solchem oder auch die ausgetragenen Flüssigkeit mittelbar erfassbar ist. Dabei kann Flüssigkeitskontakt einer entsprechenden Sensorik vermieden werden. Eine Betätigung des Spenders, die nicht zu einem Flüssigkeitsaustrag geführt hat und daher auch nicht zum Einströmen von Luft führt, führt richtigerweise nicht zu einer Erfassung.

Eine Erfassungseinrichtung ist in allgemeinster Form ein Mechanismus, der seinen Zustand reproduzierbar ändert, wenn ein Ereignis, vorliegend das Einsaugen von Luft, stattfindet. Wenngleich hier auch rein mechanische Bauweisen denkbar erscheinen, ist bei einem erfindungsgemäßen Spender vorzugsweise eine elektronische Erfassungseinrichtung vorgesehen, also eine Erfassungseinrichtung mit einem Sensor und einem Speicher, der in Folge des sensorisch mittelbar erfassten Austragsvorgangs, seinen Speicherzustand ändert. Ein Sensor ist im oder am Rande des Belüftungskanals angeordnet und erfasst den hierdurch in den Flüssigkeitsspeicher gerichteten Luftstrom unmittelbar und damit mittelbar den zuvor stattgefundenen Austrag von Flüssigkeit.

Es kommen verschiedene Sensoren in Frage, um das Einströmen der Luft zu erfassen.

So kann die Erfassungseinrichtung eine Sensoranordnung zur Erfassung der durch den Belüftungskanal einströmenden Luft aufweisen, die zur Erfassung eines Differenzdrucks zwischen zwei örtlich voneinander beabstandeten Referenzstellen des Belüftungskanals ausgebildet ist. Dies kann insbesondere erfolgen, indem im Belüftungskanal oder einem besonderen Teil davon (Messkanal) ein Bypass-Stück vorgesehen ist, welches mittels eines auslenkbaren Flächenabschnitt verschlossen ist. In Abhängigkeit des Differenzdrucks wird diese Membran ausgelenkt, was elektronisch erfassbar ist. Auch kann ein auslenkbarer Flächenabschnitt unmittelbar einen Wandungsteil des Belüftungskanals bilden und in Abhängigkeit des darin herrschenden Drucks ausgelenkt werden. Eine weitere im Kontext der Erfindung nutzbare Gestaltung sieht vor, dass die Sensoranordnung mindestens zwei Temperatursensoren sowie mindestens ein Heizelement umfasst, die im Belüftungskanal angeordnet sind, wobei die beiden Temperatursensoren stromaufwärts und stromabwärts des Heizelements vorgesehen sind. Aus der Temperaturdifferenz der Temperatursensoren und der Leistung des Heizelements kann die Erfassungseinrichtung auf die eingeströmte Luftmenge schließen.

Eine mögliche Gestaltung des Spenders und seiner Erfassungseinrichtung sehen vor, dass die Erfassungseinrichtung mittelbar über die durch den Belüftungskanal einströmende Luft die Menge der zuvor ausgetragenen Flüssigkeit ermittelt und auf einer Anzeigeeinrichtung die ermittelte ausgetragene Flüssigkeitsmenge oder die hieraus berechnete im Flüssigkeitsspeicher verbleibende Flüssigkeitsmenge anzeigt. Die Anzeigeeinrichtung kann als Display ausgestaltet sein, beispielsweise als LC-Display. Es kann je nach Anwendungszweck jedoch eine einfache LED ausreichen, insbesondere wenn die Anzeigeeinrichtung lediglich anzeigen soll, ob die ermittelte ausgetragene Flüssigkeitsmenge einer vorbestimmten Flüssigkeitsmenge entspricht bzw. diese übersteigt oder hinter dieser zurückbleibt.

Eine andere Gestaltung sieht vor, dass die Erfassungseinrichtung dafür ausgebildet ist, mittelbar über die durch den Belüftungskanal einströmende Luft Austragvorgänge als solche zu erkennen, wobei es hierfür nicht erforderlich ist, dass zwingend auch die ausgetragene Flüssigkeitsmenge mengenmäßig erfasst wird. Eine solche Gestaltung einer Erfassungseinrichtung und deren Sensorik sind technisch einfacher zu gestalten, jedoch je nach Anwendungsfeld ausreichend.

So kann bei einem Spender, der baulich bedingt immer gleich große Flüssigkeitsdosen abgibt, bereits die Information, dass ein Austrag stattgefunden hat, für eine Zählung ausreichen. Auch kann eine solche Erfassungseinrichtung in Verbindung mit einer integrierten Uhr dafür ausgebildet sein, geplante Nutzungszeitpunkte und erfolgte Austragvorgänge zu vergleichen und bei ausbleibenden Austragvorgängen zu geplanten Nutzungszeitpunkten über eine Signalisierungseinrichtung ein vorzugsweise akustisches Warnsignal abzugeben.

Die beschriebene Erfassungseinrichtung kann unmittelbar in einen Austragkopf eines Spenders integriert sein. Dies ist bei Einweg-Spendern jedoch aus ökologischen und wirtschaftlichen Gründen meist vermeidenswert. Bevorzugt werden stattdessen die Anordnung in einem externen Modul und die Anordnung in einer erfindungsgemäß vorgesehenen Schutzkappe, wie im Weiteren noch erläutert wird.

Zunächst zur Anordnung der Erfassungseinrichtung in einem externen Erfassungsmodul.

Ein solches externes Erfassungsmodul kann am Gehäuse des Spenders vorzugsweise werkzeuglos angebracht und hiervon vorzugsweise werkzeuglos gelöst werden. Je nach Anwendungszweck kann jedoch auch eine nur mittels Werkzeug ermöglichte Anbringbarkeit ausreichend sein. Das Modul stellt jedoch auch bei fester Anbringung am Spender keinen integralen Teil desselben dar. Dies bedeutet, dass das Modul entfernbar ist, ohne die Funktionalität des Spenders zum Austrag von Flüssigkeit zu verhindern.

Das externe Erfassungsmodul kann derart am Gehäuse des Spenders angebracht werden, dass es dort fest sitzt und sich nicht mehr unbeabsichtigt lösen kann. Wenn das externe Modul durch Endkunden gehandhabt werden soll, ist eine einfache Handhabung von Vorteil. Vorzugsweise wird das externe Modul in diesem Falle kraftschlüssig auf das Gehäuse aufgeschoben oder formschlüssig gegen Abziehen gesichert, insbesondere mittels eines Gewindes.

Das Erfassungsmodul ist zur externen Ankoppelung an jener Stelle des Gehäuses des Spenders ausgebildet, an dem eine Einströmöffnung in den spenderseitigen Teil des Belüftungskanals vorgesehen ist.

Das Erfassungsmodul selbst weist einen Messkanal und an dessen Ende einen Kopplungsausgang auf, wobei der Kopplungsausgang derart positioniert ist, dass der Messkanal nach Anbringung des Erfassungsmoduls kommunizierend mit der Einströmöffnung am Gehäuse des Belüftungskanals des Spenders gekoppelt ist. Vorzugsweise begrenzen eine Außenseite des Gehäuses und eine Innenseite des Erfassungsmoduls gemeinsam einen umlaufenden Kanal, mit dem sowohl die Einströmöffnung als auch der Kopplungsausgang kommunizierend verbunden sind. Nach Ankoppelung bildet der Messkanal einen Teil des Belüftungskanals.

Das Erfassungsmodul weist vorzugweise, jedoch nicht zwingend, ein ringförmiges Modulgehäuse auf, welches eine zentrische Aussparung umgibt, so dass es das Gehäuse umgebend auf dieses aufschiebbar ist.

Die Gestaltung der Erfassungseinrichtung als Teil eines externen Erfassungsmoduls ist baulich meist von Vorteil gegenüber einer integrierten Lösung in das Gehäuse des Spenders. Es gestattet die Herstellung und/oder die Verwendung des Spenders in einer Gestaltung mit und einer Gestaltung ohne Erfassungseinrichtung, ohne dass hiervon die für die Flüssigkeitsaustrag erforderlichen Spenderkomponenten und insbesondere das Gehäuse besonders gestaltet sein müssen. Zudem können sogar bereits heute am Markt befindliche Spender mit einem Erfassungsmodul nachgerüstet werden, sofern die Einströmöffnung an einer Stelle des Gehäuses vorgesehen ist, welches sich zur Ankopplung eines Erfassungsmoduls eignet.

Weiterhin kann ein externes Erfassungsmoduls einfach und insbesondere vorzugsweise werkzeuglos von einem entleerten Einweg-Spender entfernt und auf einen neuen Spender aufgesetzt werden, was ökonomisch und ökologisch vorteilhaft ist. Eine besondere Ausgestaltung des Erfassungsmoduls kann daher mit Sensorik ausgebildet sein, um einen solchen Spenderwechsel zu registrieren und diese Information für einen Rücksetzvorgang zu nutzen. Beispielsweise kann ein Zähler auf Null zurückgesetzt werden.

Wie oben bereits erwähnt, ist auch die Gestaltung einer erfindungsgemäßen Schutzkappe mit integrierter Erfassungseinrichtung vorteilhaft. Bevor hierauf im Detail eingegangen wird, wird zunächst die Gestaltung eines erfindungsgemäßen Spenders gemäß der Erfindung erläutert, wobei diese Gestaltung insbesondere als Grundlage für die Verwendung einer Schutzkappe mit Erfassungseinrichtung dient.

Gemäß der erfindungsgemäßen Gestaltung ist ein gattungsgemäßer Spender mit einer Schutzkappe zur Überdeckung der Austragöffnung im aufgesetzten Zustand ausgestattet. Die Schutzkappe wird vom Benutzer vor Benutzung des Spenders abgenommen und anschließend wieder aufgesetzt. Die kann insbesondere eine Steckkappe oder eine Schraubkappe sein.

Der Spender gemäß der Erfindung weist im Belüftungskanal ein Schaltventil auf, welches über Betätigung einer an der Außenseite des Gehäuses vorgesehenen Schaltfläche geöffnet werden kann. Der Belüftungskanal ist somit bei nicht betätigtem Schaltventil geschlossen. Ein Einsaugen von Luft wird unterbunden. Das Schaltventil ist vorzugsweise im Bereich einer außenseitigen Oberfläche des Gehäuses des Spenders vorgesehen, so dass es den Eintritt von Luft in den Belüftungskanals unterbindet.

Zur Betätigung der genannten Schaltfläche ist vorgesehen, dass das Gehäuse und die Schutzkappe derart aufeinander abgestimmt sind, dass die Schutzkappe im auf das Gehäuse aufgesetzten Zustand oder beim Aufsetzen auf die Schaltfläche wirkt und so das Schaltventil öffnet. Dies bedeutet, dass der Vorgang des Aufsetzens der Schutzkappe zumindest zwischenzeitlich ein Öffnen des Schaltventils bewirkt und damit ein Einströmen von Luft gestattet.

Es ist bei einer solchen Gestaltung des Spenders somit nicht vorgesehen, dass alleine der Flüssigkeitsaustrag und der sich dadurch einstellende Unterdruck im Flüssigkeitsspeicher das Einsaugen von Luft ermöglicht. Stattdessen bleibt der Unterdruck zunächst erhalten und wird ggf. durch mehrfaches Betätigen des Spenders und dementsprechend mehrfachen Austrag von Flüssigkeit noch verstärkt. Erst wenn die Schutzkappe wieder auf das Gehäuse aufgesetzt wird, kann die Belüftung erfolgen.

Dies hat mehrere Vorteile. Unabhängig vom Vorhandensein einer Erfassungseinrichtung können ein tendenziell stärkerer Unterdruck und ein dadurch verursachter größerer Luftstrom der einströmenden Luft von Vorteil sein, beispielsweise um Flüssigkeitsreste von einem Flüssigkeitsfilter am Ende des Bellüftungskanals zu lösen.

Von besonderem Vorteil ist eine derartige Ausgestaltung eines Spenders jedoch insbesondere, wenn dieser eine Erfassungseinrichtung oben beschriebener Art aufweist. Eine solche Erfassungseinrichtung ist hinsichtlich ihrer Sensorik technisch einfacher zu gestalten, wenn ein größerer Luftstrom erfasst wird, wie er durch die zwischenzeitliche Blockierung des Belüftungskanals mittels des Schaltventils erreichbar ist.

Insbesondere von Vorteil ist es, wenn die Erfassungseinrichtung in die Schutzkappe integriert ist. Hierdurch ergeben sich die oben bereits in Hinblick auf das externe Erfassungsmodul thematisierten Vorteile, insbesondere die Wiederverwendbarkeit und die Möglichkeit, Spender mit und ohne Erfassungseinrichtung bei hoher baulicher Übereinstimmung anbieten zu können.

Im Falle der Integration der Erfassungseinrichtung in die Schutzkappe ist diese vorzugsweise gestaltet, wie es ebenfalls zum externen Erfassungsmodul beschrieben ist, also mit einem Messkanal, in oder an dem die Sensorik vorgesehen ist und der durch das Aufsetzen der Schutzkappe zum Teil des Belüftungskanals wird. Die an der Schutzkappe vorgesehene Betätigungsfläche zur Betätigung der Schaltfläche des Schaltventils ist vorzugsweise derart angeordnet, dass der Messkanal des Schutzkappe und der Belüftungskanal im Gehäuse kommunizierend und im Übergangsbereich dicht gegenüber einer Umgebung verbunden sind, so dass die kappenseitige Erfassungseinrichtung den Luftstrom unmittelbar erfassen kann, sobald das Schaltventil beim Aufsetzen mittels der Schutzkappe geöffnet wird.

Die Erfassung der einströmenden Luft im Zuge des Aufsetzens der Schutzkappe kann auch deshalb von Vorteil sein, da sie nur eine kurzzeitige Phase benötigt, in der die Elektronik der Erfassungseinrichtung aktiviert ist. Es ist daher insbesondere von Vorteil, wenn die Erfassungseinrichtung über einen Schalter verfügt, der durch das Aufsetzen der Schutzkappe auf das Gehäuse betätigbar ist.

Das Auslösen dieses Schalters, der bei einer Erfassungseinrichtung innerhalb des Gehäuses des Spenders auch mit dem Schaltventil eine gemeinsame Baueinheit bilden kann, aktiviert die Erfassungseinrichtung. Sobald der dann anschließend sensierte Luftstrom abgeschlossen ist und ausgewertet wurde, kann die Erfassungsrichtung sich wieder deaktivieren. Es wird somit nur wenig elektrische Energie verbraucht. Eine Batterie als Teil der Erfassungseinrichtung kann daher für eine lange Zeit, ggf. mehrere Jahre, Energie für den Betrieb der Erfassungseinrichtung bieten.

### KURZBESCHREIBUNG DER ZEICHNUNGEN

Weitere Vorteile und Aspekte der Erfindung ergeben sich aus den Ansprüchen und aus der nachfolgenden Beschreibung eines bevorzugten Ausführungsbeispiels der Erfindung, das nachfolgend anhand der Figuren erläutert sind.
Fig. 1 sowie 2A und 2B zeigen einen an sich bekannten Spender als Ausgangspunkt für die anschließend beschriebenen erfindungsgemäßen Gestaltungen sowie die grundsätzliche Funktionsweise dieses Spenders.
Fig. 3 zeigt ein nicht von der Erfindung umfasstes Ausführungsbeispiel mit in den Spender integrierter Erfassungseinrichtung.
Fig. 4A und 4B zeigen ein nicht von der Erfindung umfasstes Ausführungsbeispiel mit einem externen Erfassungsmodul.
Fig. 5A und 5B zeigen ein Ausführungsbeispiel der Erfindung mit einem in eine Schutzkappe integrierten Erfassungsmodul.

### DETAILLIERTE BESCHREIBUNG DER AUSFÜHRUNGSBEISPIELE

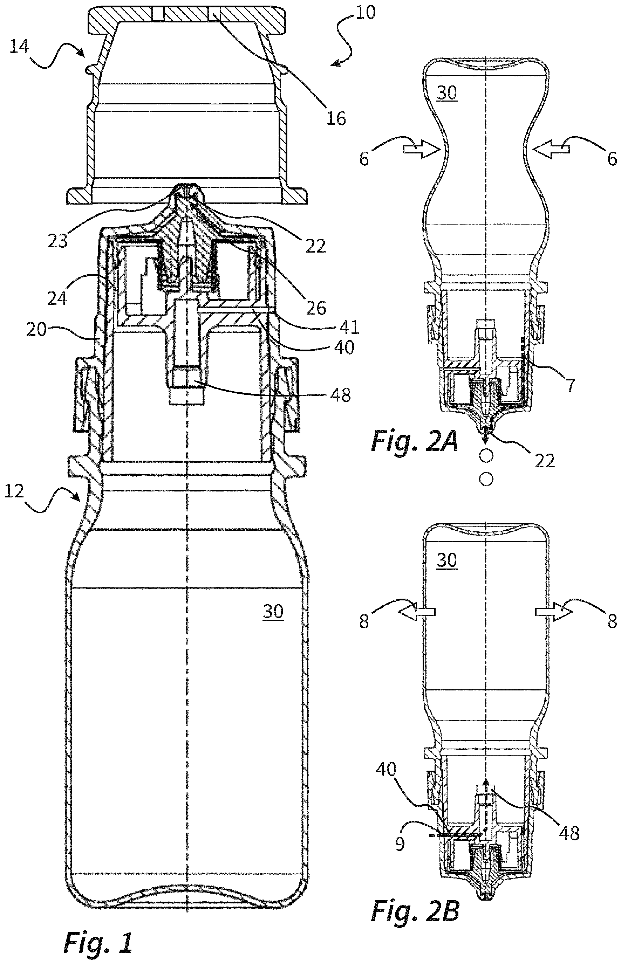

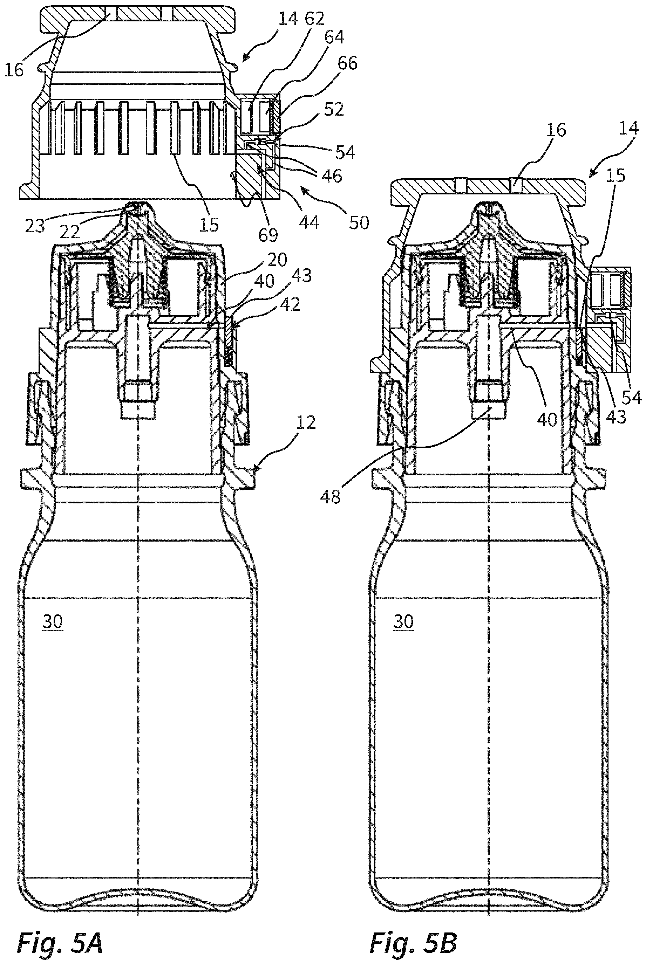

Die Fig. 1 sowie 2A und 2B zeigen zunächst einen gattungsgemäßen Flüssigkeitsspender, der im Weiteren gemäß der Gestaltung der Fig. 5A und 5B durch Modifikation oder Ergänzung zu einem erfindungsgemäßen Flüssigkeitsspender weitergebildet wird.

Der Flüssigkeitsspender 10 verfügt über eine Austragvorrichtung 12, die die Hauptkomponente des Flüssigkeitsspenders 10 darstellt und ihrerseits über ein Gehäuse 20 verfügt, welches am distalen Ende von einer Austragöffnung 22 durchbrochen ist. Zum Schutz dieser Austragöffnung 22 umfasst der Spender 10 eine Schutzkappe 14, die vorliegend als belüftete Schutzkappe ausgebildet ist, um im aufgesetzten Zustand an der Austragöffnung 22 verbliebene Flüssigkeitsreste schnell durch Belüftungslöcher 16 entweichen zu lassen.

Die Austragvorrichtung 12 verfügt über einen Flüssigkeitsspeicher 30, dessen Wandungen eine Quetschflasche darstellen, wie im Weiteren noch erläutert wird. Auf den Flüssigkeitsspeicher 30 aufgesetzt ist ein Austragkopf, welcher neben der bereits erwähnten Austragöffnung 22 einen Austragkanal 24 zur Verfügung stellt, der den Flüssigkeitsspeicher 30 mit der Austragöffnung 22 verbindet. Der Austragöffnung 22 vorgeschaltet ist ein Auslassventil 26, welches bei ausreichendem Flüssigkeitsdruck in einer Ventilkammer öffnet und somit den Austritt von Flüssigkeit durch die Austragöffnung 22 hindurch gestattet. Vorliegend ist der Spender 10 als Tropfenspender ausgebildet und verfügt jenseits der Austragöffnung 22 über eine Tropfenbildungsfläche 23, die außenseitig durch eine Abrisskante begrenzt ist.

Wie bereits erwähnt, handelt es sich beim Flüssigkeitsspeicher 30 um einen in Art einer Quetschflasche ausgebildeten Flüssigkeitsspeicher 30. Dies bedeutet, dass in der durch Fig. 2A verdeutlichten Weise die Betätigung des Spenders dadurch erfolgt, dass die Flasche in Richtung der Pfeile 6 zusammengedrückt wird. Hierdurch entsteht ein Überdruck im Flüssigkeitsspeicher 30, der bewirkt, dass Flüssigkeit entlang des Austrittspfads 7 zur Austragöffnung 22 und der Tropfenbildungsfläche 23 gefördert wird und hier in Tropfenform abgegeben wird. Entfällt die Kraftbeaufschlagung der Quetschflasche, so nimmt diese in der durch die Pfeile 8 in Fig. 2B verdeutlichten Weise wieder ihre Ausgangsform an, wodurch es zu einem Unterdruck im Flüssigkeitsspeicher 30 kommt. Dies bewirkt, dass durch einen Belüftungskanal 40 Luft entlang des Belüftungspfades 9 eingesogen wird. Die eingesogene Luft wird am Ende des Belüftungskanals 40 im Bereich einer Filteranordnung 48 von Verunreinigungen wie Keimen und Bakterien gereinigt. Die einströmende Luft sorgt für einen Druckausgleich im Flüssigkeitsspeicher 30, so dass der zwischenzeitlich bestehende Unterdruck abgebaut wird.

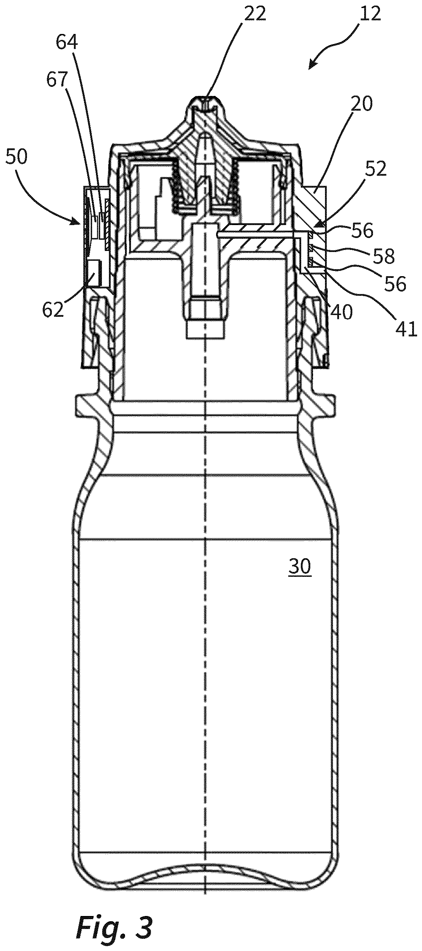

Fig. 3 zeigt eine Modifikation des Spenders der Fig. 1, vorliegend insbesondere eine Modifikation der Austragvorrichtung 12. Die Modifikation besteht darin, dass das Gehäuse 20 anders geartet ist und eine Erfassungseinrichtung 50 umgibt, die zur Erfassung von durch den Belüftungskanal 40 einströmender Luft ausgestaltet ist. Die Erfassungseinrichtung 50 verfügt über einen akustischen Signalgeber 67 in Form eines Lautsprechers, über einen Mikroprozessor 64 sowie eine Energiequelle in Form einer Batterie 62.

Weiterhin verfügt die Erfassungseinrichtung 50 über eine Sensoranordnung 52 im Belüftungskanal40, die insbesondere an dessen Wandung vorgesehen ist. Diese Sensoranordnung 52 umfasst zwei Temperatursensoren 56 sowie ein dazwischen angeordnetes Heizelement 58. Wenn nun beim Ansaugen von Luft entsprechend der Phase der Fig. 2B Luft durch den Belüftungskanal 40 strömt, so kann durch den ersten Temperatursensor 56 deren Temperatur vor dem Heizelement 58 erfasst werden und durch den zweiten Temperatursensor 56 stromabwärts des Heizelements 58 die durch das Heizelement 58 erwärmte Luft bezüglich ihrer Temperatur gemessen werden. Die Temperaturdifferenz ist ein Indikator dafür, ob und wieviel Luft durch den Belüftungskanal 40 geströmt ist. Eine hohe Temperaturdifferenz tritt bei geringem Luftstrom auf. Eine geringe Temperaturdifferenz tritt bei großem Luftstrom auf.

Das Ergebnis der Messungen dessen kann durch den Mikroprozessor 64 ausgewertet werden. So kann der Mikroprozessor 64 beispielsweise bei einem zu geringen Luftstrom und einem demzufolge zuvor zu geringen Flüssigkeitsaustrag über den Lautsprecher 67 zu erkennen geben, dass der Austrag nicht ordnungsgemäß stattgefunden hat. Auch kann der Mikroprozessor 64, wenn in seinem Speicher vorgegebene Abgabezeitpunkte hinterlegt sind, über den Lautsprecher 67 zu erkennen geben, wenn ein geplanter Austrag nicht stattgefunden hat. Der Benutzer wird somit daran erinnert, diesen Austrag nachzuholen.

Die Anordnung der Sensoranordnung 52 im Belüftungskanal 40 führt dazu, dass ein Austrag sicher erkannt werden kann, ohne dass die Sensoranordnung hierfür unmittelbar in Flüssigkeitskontakt stehen muss.

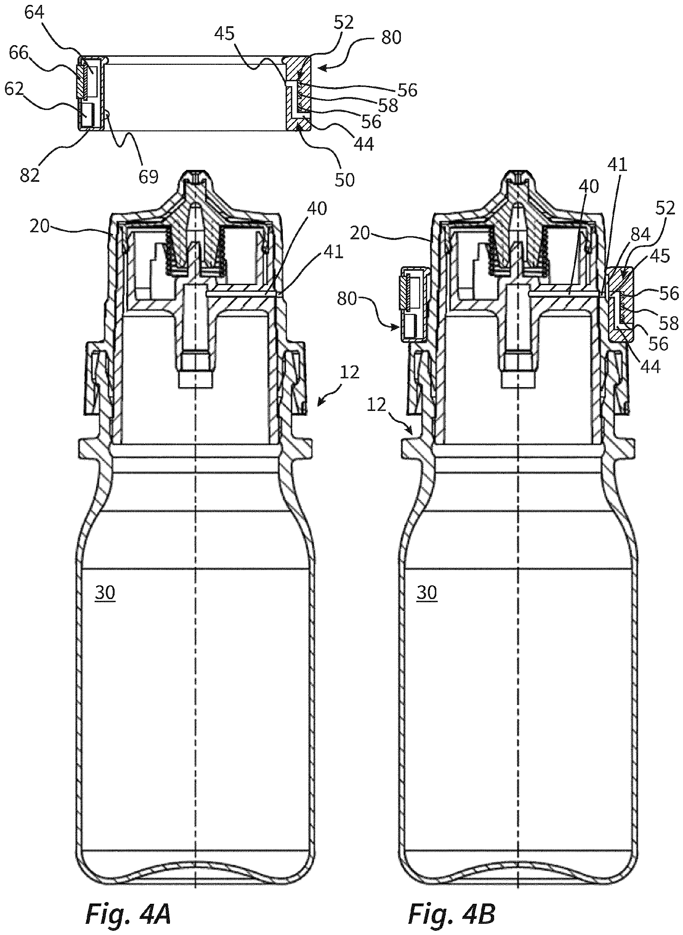

Bei der Ausgestaltung gemäß den Fig. 4A und 4B ist das Grundprinzip ähnlich jenem der Fig. 3. Wie anhand der Fig. 4A ersichtlich ist, ist die Erfassungseinrichtung 50 vorliegend jedoch als Teil eines Erfassungsmoduls 80 ausgestaltet, welches über ein ringförmiges Modulgehäuse 82 verfügt, das zum Aufschieben auf das Gehäuse 20 der Austragvorrichtung 12 vorgesehen ist. Fig. 4B zeigt den aufgeschobenen Zustand.

Das Erfassungsmodul 80 umfasst ebenfalls eine Batterie 62 sowie einen Mikroprozessor 64. Bei dieser Gestaltung ist jedoch zusätzlich ein LC-Display 66 vorgesehen, welches die Vermittlung komplexerer Informationen an den Benutzer ermöglicht, als dies üblicherweise mit einem Lautsprecher entsprechend der Fig. 3 der Fall ist. Allerdings kann auch bei der Gestaltung der Fig. 3 und der dort vorgesehen Integration der Erfassungseinrichtung 50 in das Gehäuse 20 statt des Lautsprechers oder zusätzlich ein Display vorgesehen sein.

Die Sensoranordnung 52 ist beim Ausführungsbeispiel der Fig. 4A und 4B jener der Fig. 3 ähnlich und basiert wiederum auf dem Vorhandensein zweier Temperatursensoren 56 und eines Heizelements 58. In diesem Falle ist die Sensoranordnung 52 jedoch nicht entsprechend der Fig. 3 integraler Bestandteil der Austragvorrichtung 12, sondern Teil des erwähnten Erfassungsmoduls 80. Bezug nehmend auf Fig. 4A ist ersichtlich, dass an der Innenseite des ringförmigen Modulgehäuses 82 des Erfassungsmoduls 80 ein nur schematisch dargestellter Schalter 69 vorgesehen ist. Das Erfassungsmodul kann mittels dieses Schalters erkennen, wenn es auf das Gehäuse 20 der Austragvorrichtung 12 aufgeschoben wird. Dies kann beispielsweise genutzt werden, um einen integrierten Zähler auf einen Ausgangswert zurückzusetzen. Beim Montieren des Erfassungsmoduls 80 wird dieser Schalter 69 ausgelöst.

Fig. 4B zeigt den montierten Zustand. Hieran ist ersichtlich, dass ein Kopplungsausgang 45 des Messkanals 44 des Erfassungsmoduls 80 in kommunizierender Verbindung mit einer Einströmöffnung 41 des austragsvorrichtungsseitigen Teils des Belüftungskanals 40 steht. Um eine nicht fluchtende Ausrichtung ebenfalls zu gestatten, sind das Gehäuse 20 der Austragvorrichtung 12 sowie das Modulgehäuse 82 des Erfassungsmoduls 80 dafür ausgebildet, im gekoppelten Zustand einen Ringraum 84 zu definieren. Dies gestattet es, das Erfassungsmodul in beliebiger Stellung auf das Gehäuse 20 aufzuschieben.

Wie bereits erwähnt, ist die Sensoranordnung 52 in gleicher Art und Weise wie beim Ausführungsbeispiel der Fig. 3 dargestellt. Das Vorhandensein des LC-Displays 66 gestattet es jedoch, die über diese Sensoranordnung 52 erfassten Werte in noch weitergehender Art auszuwerten und dem Benutzer zur Verfügung zu stellen. So kann insbesondere durch das Erfassen des Luftstroms und der Dauer des Luftstroms beim Einsaugen von Luft durch den Messkanal44 und den Belüftungskanal40 abgeschätzt werden, welche Luftmenge in die Flasche eingetreten ist und demzufolge welche Flüssigkeitsmenge zuvor ausgetragen wurde. Auf dem LC-Display 66 kann dementsprechend die insgesamt bereits ausgetragene Flüssigkeitsmenge oder im Falle, dass das Erfassungsmodul 80 die Ausgangsmenge kennt, auch die verbleibende Flüssigkeitsmenge im Flüssigkeitsspeicher 30 dargestellt werden.

Die Ausgestaltung der Fig. 5A und 5B ähnelt jener der Fig. 4A und 4B im Hinblick darauf, dass auch hier die Erfassungseinrichtung 50 nicht integraler Bestandteil der Austragvorrichtung 12 ist. Allerdings ist die Erfassungseinrichtung 50 in diesem Falle in der Schutzkappe 14 vorgesehen. Auch diese Erfassungseinrichtung verfügt über eine Batterie 62, einen Mikroprozessor 64 sowie ein LC-Display 66. Die Sensoranordnung 52, die an einem Messkanal 44 der Kappe 14 vorgesehen ist, ist vorliegend jedoch anders gestaltet, wobei es grundsätzlich willkürlich ist, welches der Ausführungsbeispiele mit welcher Art der Sensoranordnung 52 ausgestaltet ist. Die Sensoranordnung 52 der Fig. 5A und 5B verfügt über einen zweiteiligen Seitenkanal 46, der vom Messkanal 44 abgeht und in dem eine Membran 54 angeordnet ist. Abhängig von dem Luftstrom, der durch den Messkanal 44 strömt, wird diese Membran 54 in unterschiedlichem Maße ausgelenkt, was beispielsweise mittels eines Piezosensors erfassbar und durch den Mikroprozessor 64 weiterverarbeitbar ist.

Die Schutzkappe 14 verfügt darüber hinaus ebenfalls über einen Schalter 69, der jedoch eine etwas andere technische Bedeutung hat als der Schalter 69 im Falle der Fig. 4A und 4B, wie im Weiteren noch erläutert wird.

Da die Schutzkappe während des Austrags von Flüssigkeit naturgemäß nicht auf der Austragvorrichtung 12 angebracht ist, verfügt die Austragvorrichtung 12 über ein Schaltventil 42, welches die Einströmöffnung des Belüftungskanals 40 im Zustand der Fig. 5A überdeckt und somit das Einströmen von Luft zu diesem Zeitpunkt nicht gestattet. Wenn der Spender der Fig. 5A somit wie in Fig. 2A dargestellt betätigt wird, kommt es zwar zum Flüssigkeitsaustrag, zunächst jedoch nicht zu einem Druckausgleich durch einströmende Luft. Vielmehr baut sich im Falle eines mehrfachen Austrags ein immer weiter ansteigender Unterdruck im Flüssigkeitsspeicher 30 auf, der zu diesem Zeitpunkt noch nicht ausgeglichen werden kann.

Erst wenn in der durch Fig. 5B verdeutlichten Weise die Schutzkappe 14 auf die Austragvorrichtung 12 aufgesetzt wird, kann es zu einer Erfassung kommen. Zunächst wird der Schalter 69 ausgelöst, was zu einer Aktivierung der Erfassungseinrichtung 50 führt. Wird die Kappe bis in ihre Endlage der Fig. 5B niedergedrückt, so können Betätigungsflächen 15, die umlaufend am unteren Ende von Rippen an der Schutzkappe 14 vorgesehen sind, auf eine Schaltfläche 43 des Schaltventils 42 drücken und dieses dadurch in der durch Fig. 5B verdeutlichten Weise niederdrücken. Hierdurch wird der Belüftungskanal 40 geöffnet, allerdings erst, nachdem die Schutzkappe 14 so weit aufgesetzt ist, dass weder an ihrem unteren Rand entlang noch durch die Belüftungsöffnungen an der Stirnfläche der Kappe Luft einströmen kann. Dies bedeutet, dass der bei hohem Unterdruck recht schlagartige Druckausgleich durch den Messkanal 44 der kappenseitigen zuvor per Schalter 69 aktivierten Erfassungseinrichtung 50 erfolgt.

Der Mikroprozessor 64 der Erfassungseinrichtung 50 kann dann auf dem LC-Display 66 Daten hierzu anzeigen, insbesondere die bislang insgesamt ausgetragene Flüssigkeitsmenge oder aber die noch verbleibende Flüssigkeitsmenge im Flüssigkeitsspeicher 30. Der schlagartige Druckausgleich ist darüber hinaus auch von Vorteil, um möglicherweise noch an der Filteranordnung 48 verbliebene Flüssigkeitsreste von dieser zu trennen.

Sobald die Sensoranordnung 52 keinen Luftstrom im Messkanal 44 mehr wahrnimmt, kann sie in einen Stromsparmodus wechseln oder sich komplett deaktivieren, bis nächstmalig der Schalter 69 ausgelöst wird. Selbst bei einer nur kleinen Batterie 62 kann die Schutzkappe 14 dadurch lange ihre Funktion als den Austrag protokollierende Schutzkappe übernehmen.

## Patentansprüche

1. Spender (10) zum Austrag von Flüssigkeit, insbesondere zum Austrag einer pharmazeutischen Flüssigkeit, mit den folgenden Merkmalen:
a. der Spender (10) weist ein Gehäuse (20) mit einer Austragöffnung (22) zum Austrag von Flüssigkeit auf, und
b. der Spender (10) weist einen Flüssigkeitsspeicher (30) zur Lagerung der Flüssigkeit vor dem Austrag auf, und
c. der Spender (10) weist einen Belüftungskanal (40) auf, mittels dessen eine umgebende Atmosphäre mit dem Flüssigkeitsspeicher (30) verbunden ist, um nach dem Austrag von Flüssigkeit ein Einströmen von Luft in den Flüssigkeitsspeicher (30) zu gestatten, und
d. der Spender (10) weist eine Schutzkappe (14) zur Überdeckung der Austragöffnung (22) auf, die auf das Gehäuse (20) aufsetzbar und vom Gehäuse (20) abnehmbar ist, **gekennzeichnet durch** die folgenden Merkmale:
e. der Spender (10) weist im Belüftungskanal (40) ein Schaltventil (42) auf, welches über Betätigung einer an der Außenseite des Gehäuses vorgesehenen Schaltfläche (43) geöffnet werden kann, und
f. das Gehäuse (20) und die Schutzkappe (14) sind derart aufeinander abgestimmt, dass die Schutzkappe (14) im auf das Gehäuse (20) aufgesetzten Zustand oder beim Aufsetzen der Schutzkappe (14) auf die Schaltfläche (43) wirkt und so das Schaltventil (42) öffnet.

2. Spender (10) nach Anspruch 1 mit den folgenden zusätzlichen Merkmalen:
a. der Spender (10) weist eine Erfassungseinrichtung (50) zur Erfassung eines Austragvorgangs auf, und
b. die Erfassungseinrichtung (50) ist zur Erfassung der durch den Belüftungskanal (40) einströmenden Luft ausgebildet.

3. Spender (10) nach Anspruch 2 mit dem folgenden zusätzlichen Merkmal:
a. die Erfassungseinrichtung (50) weist eine Sensoranordnung (52) zur Erfassung der durch den Belüftungskanal (40) einströmenden Luft auf, wobei
- die Sensoranordnung (52) zur Erfassung eines Differenzdrucks zwischen zwei örtlich voneinander beabstandeten Referenzstellen des Belüftungskanals ausgebildet ist, und/oder
- die Sensoranordnung (52) mindestens einen auslenkbaren Flächenabschnitt (54) aufweist, der durch den Belüftungskanal durchströmende Luft auslenkbar ist, und/oder
- die Sensoranordnung (52) mindestens zwei Temperatursensoren (56) sowie mindestens ein Heizelement (58) umfasst, die im Belüftungskanal (40) angeordnet sind, wobei die beiden Temperatursensoren (56) stromaufwärts und stromabwärts des Heizelements (58) vorgesehen sind.

4. Spender (10) nach einem der Ansprüche 2 oder 3 mit den folgenden zusätzlichen Merkmalen:
a. die Erfassungseinrichtung (50) ist dafür ausgebildet, mittelbar über die durch den Belüftungskanal (40) einströmende Luft die Menge der zuvor ausgetragenen Flüssigkeit zu ermitteln, und
b. die Erfassungseinrichtung (50) weist eine Anzeigeeinrichtung (66) auf und ist dafür ausgebildet, die ermittelte ausgetragene Flüssigkeitsmenge oder die hieraus berechnete im Flüssigkeitsspeicher (30) verbleibende Flüssigkeitsmenge über die Anzeigeeinrichtung (66) einem Benutzer zur Kenntnis zu bringen.

5. Spender (10) nach einem der Ansprüche 2 bis 4 mit dem folgenden weiteren Merkmal:
a. die Erfassungseinrichtung (50) ist dafür ausgebildet, mittelbar über die durch den Belüftungskanal (40) einströmende LuftAustragvorgänge zu erkennen,
vorzugsweise mit dem zusätzlichen Merkmal:
b. die Erfassungseinrichtung (50) verfügt über eine interne Uhr und ist dafür ausgebildet, geplante Nutzungszeitpunkte und erfolgte Austragvorgänge zu vergleichen und bei ausbleibenden Austragvorgängen zu geplanten Nutzungszeitpunkten über eine Signalisierungseinrichtung (67) ein Warnsignal abzugeben.

6. Spender (10) nach einem der Ansprüche 2 bis 5 mit mindestens einem der folgenden weiteren Merkmale:
a. der Spender (10) ist als Quetschflaschenspender ausgebildet und verfügt über einen manuell von außen zum Zwecke des Flüssigkeitsaustrags komprimierbaren Flüssigkeitsspeicher (30), und/oder
b. der Flüssigkeitsspeicher (30) weist ein Maximalvolumen zwischen 5 ml und 500 ml auf, vorzugweise zwischen 5 ml und 50 ml, und/oder
c. der Spender (10) ist als Tropfenspender (10) ausgebildet und verfügt im Bereich der Austragöffnung (22) über eine Tropfenbildungsfläche (23), die insbesondere vorzugsweise durch eine Abrisskante außenseitig begrenzt ist, und/oder
d. der Spender ist als pharmazeutischer Spender (10) ausgebildet und weist einen mit einer pharmazeutischen Flüssigkeit befüllten Flüssigkeitsspeicher (30) auf, insbesondere mit einer pharmazeutischen Flüssigkeit zur ophthalmischen Applikation.

7. Spender (10) nach einem der Ansprüche 2 bis 6 mit dem folgenden zusätzlichen Merkmal:
a. die Erfassungseinrichtung (50) ist in Form eines externen Erfassungsmoduls (80) ausgebildet, welches am Gehäuse (20) des Spenders (10) vorzugsweise werkzeuglos angebracht und hiervon vorzugsweise werkzeuglos gelöst werden kann,
insbesondere vorzugsweise mit dem zusätzlichen Merkmal:
b. das Erfassungsmodul (80) verfügt über einen Schalter (69), der durch das Befestigen des Erfassungsmoduls (80) auf das Gehäuse (20) betätigbar ist.

8. Spender (10) zum Austrag von Flüssigkeit nach einem der Ansprüche 2 bis 6 mit dem folgenden weiteren Merkmal:
a. die Erfassungseinrichtung (50) ist in die Schutzkappe (14) integriert.

9. Spender (10) zum Austrag von Flüssigkeit nach einem der vorstehenden Ansprüche mit dem folgenden weiteren Merkmal:
a. die Erfassungseinrichtung (50) verfügt über einen Schalter (69), der durch das Aufsetzen der Schutzkappe (14) auf das Gehäuse (20) betätigbar ist.

10. Spender zum Austrag von Flüssigkeit nach einem der vorstehenden Ansprüche mit dem folgenden weiteren Merkmal:
a. die Erfassungseinrichtung (50) ist dafür ausgebildet, die Luftströmung im Belüftungskanal (40) in Reaktion auf ein Aufsetzen der Schutzkappe (14) zu ermitteln.

## Claims

1. Dispenser (10) for delivering liquid, in particular for delivering a pharmaceutical liquid, having the following features:
a. the dispenser (10) has a housing (20) with a delivery opening (22) for delivering liquid; and
b. the dispenser (10) has a liquid reservoir (30) for storing the liquid prior to delivery; and
c. the dispenser (10) has a ventilation duct (40) by means of which a surrounding atmosphere is connected to the liquid reservoir (30) so as to permit air to flow into the liquid reservoir (30) after liquid has been delivered; and
d. the dispenser (10) has a protective cap (14) for covering the delivery opening (22), said protective cap (14) being able to be placed onto the housing (20) and removed from the housing (20),
**characterized by** the following features:
e. the dispenser (10) has a switch valve (42) in the ventilation duct (40), said switch valve (42) being able to be opened by actuating a pushbutton (43) provided on the external side of the housing; and
f. the housing (20) and the protective cap (14) are mutually adapted in such a manner that the protective cap (14) in the state placed onto the housing (20), or when placing the protective cap (14) on the latter, acts on the pushbutton (43) and in this way opens the switch valve (42).

2. Dispenser (10) according to Claim 1, having the following additional features:
a. the dispenser (10) has a detection device (50) for detecting a delivery procedure; and
b. the detection device (50) is configured to detect the air flowing in through the ventilation duct (40).

3. Dispenser (10) according to Claim 2, having the following additional feature:
a. the detection device (50) has a sensor assembly (52) for detecting the air flowing in through the ventilation duct (40); wherein
- the sensor assembly (52) is configured to detect a pressure difference between two physically spaced apart reference locations of the ventilation duct; and/or
- the sensor assembly (52) has at least one deflectable surface portion (54) which is deflectable by air flowing through the ventilation duct; and/or
- the sensor assembly (52) comprises at least two temperature sensors (56) as well as at least one heating element (58) which are disposed in the ventilation duct (40), wherein the two temperature sensors (56) are provided upstream and downstream of the heating element (58).

4. Dispenser (10) according to one of Claims 2 or 3, having the following additional features:
a. the detection device (50), by way of the air flowing in through the ventilation duct (40), is configured to determine indirectly the quantity of liquid previously delivered; and
b. the detection device (50) has a display device (66) and, by way of the display device (66), is configured to inform a user about the determined quantity of liquid delivered, or the quantity of liquid remaining in the liquid reservoir (30) calculated therefrom.

5. Dispenser (10) according to one of Claims 2 to 4, having the following further feature:
a. the detection device (50), by way of the air flowing in through the ventilation duct (40), is configured to identify indirectly delivery procedures;
preferably having the additional feature:
b. the detection device (50) possesses an internal clock and is configured to compare planned utilization times and performed delivery procedures and, in the event of delivery procedures being absent at the planned utilization times, to emit a warning signal by way of a signalling device (67).

6. Dispenser (10) according to one of Claims 2 to 5, having at least one of the following further features:
a. the dispenser (10) is configured as a squeeze bottle dispenser and possesses a liquid reservoir (30) which for the purpose of delivering liquid is manually compressible from the outside; and/or
b. the liquid reservoir (30) has a maximum volume between 5 ml and 500 ml, preferably between 5 ml and 50 ml; and/or
c. the dispenser (10) is configured as a drop dispenser (10) and in the region of the delivery opening (22) possesses a drop formation face (23) which particularly preferably is externally delimited by a separation edge; and/or
d. the dispenser is configured as a pharmaceutical dispenser (10) and has a liquid reservoir (30) which is filled with a pharmaceutical liquid, in particular with a pharmaceutical liquid for ophthalmic application.

7. Dispenser (10) according to one of Claims 2 to 6, having the following additional feature:
a. the detection device (50) is configured in the form of an external detection module (80) which is preferably attached to the housing (20) of the dispenser (10) without tools, and can preferably be removed therefrom without tools;
particularly preferably having the additional feature:
b. the detection module (80) possesses a switch (69) which is activatable by fastening the detection module (80) to the housing (20).

8. Dispenser (10) for delivering liquid according to one of Claims 2 to 6, having the following further feature:
a. the detection device (50) is integrated in the protective cap (14).

9. Dispenser (10) for delivering liquid according to one of the preceding claims, having the following further feature:
a. the detection device (50) possesses a switch (69) which is activatable by placing the protective cap (14) onto the housing (20).

10. Dispenser for delivering liquid according to one of the preceding claims, having the following further feature:
a. the detection device (50), as a response to placing the protective cap (14), is configured to determine the air flow in the ventilation duct (40).

## Revendications

1. Distributeur (10) pour décharger un liquide, notamment pour décharger un liquide pharmaceutique, avec les caractéristiques suivantes :
a. le distributeur (10) présente un boîtier (20) avec un orifice de déchargement (22) pour décharger du liquide, et
b. le distributeur (10) présente un réservoir de liquide (30) pour stocker le liquide avant le déchargement, et
c. le distributeur (10) présente un canal de ventilation (40) au moyen duquel une atmosphère environnante est reliée au réservoir de liquide (30) afin de permettre à de l'air d'entrer dans le réservoir de liquide (30) après le déchargement de liquide, et
d. le distributeur (10) présente un capuchon de protection (14) pour recouvrir l'ouverture de déchargement (22), qui peut être placé sur le boîtier (20) et détaché du boîtier (20),
**caractérisé par** les caractéristiques suivantes :
e. le distributeur (10) présente une soupape de commutation (42) dans le canal de ventilation (40), qui peut être ouverte en actionnant une surface de commutation (43) prévue sur le côté extérieur du boîtier, et
f. le boîtier (20) et le capuchon de protection (14) sont adaptés l'un à l'autre de telle sorte que le capuchon de protection (14), à l'état placé sur le boîtier (20) ou lorsque le capuchon de protection (14) est mis en place, agit sur la surface de commutation (43) et ouvre ainsi la soupape de commutation (42).

2. Distributeur (10) selon la revendication 1, avec les caractéristiques supplémentaires suivantes :
a. le distributeur (10) présente un dispositif de détection (50) pour détecter une opération de déchargement, et
b. le dispositif de détection (50) est configuré pour détecter l'air entrant par le canal de ventilation (40).

3. Distributeur (10) selon la revendication 2 avec la caractéristique supplémentaire suivante :
a. le dispositif de détection (50) présente un agencement de capteurs (52) pour détecter l'air entrant par le canal de ventilation (40),
- l'agencement de capteurs (52) est configuré pour détecter une pression différentielle entre deux points de référence du canal de ventilation espacés localement l'un de l'autre, et/ou
- l'agencement de capteurs (52) présente au moins une section de surface (54) pouvant être déviée, qui peut être déviée par de l'air s'écoulant à travers le canal de ventilation, et/ou
- l'agencement de capteurs (52) comprend au moins deux capteurs de température (56) ainsi qu'au moins un élément chauffant (58), qui sont agencés dans le canal de ventilation (40), les deux capteurs de température (56) étant prévus en amont et en aval de l'élément chauffant (58).

4. Distributeur (10) selon l'une quelconque des revendications 2 ou 3, avec les caractéristiques supplémentaires suivantes :
a. le dispositif de détection (50) est configuré pour déterminer indirectement, par l'intermédiaire de l'air entrant par le canal de ventilation (40), la quantité de liquide précédemment déchargé, et
b. le dispositif de détection (50) présente un dispositif d'affichage (66) et est configuré pour porter à la connaissance d'un utilisateur, par l'intermédiaire du dispositif d'affichage (66), la quantité de liquide déchargée déterminée ou la quantité de liquide restant dans le réservoir de liquide (30) calculée à partir de celle-ci.

5. Distributeur (10) selon l'une quelconque des revendications 2 à 4, avec la caractéristique supplémentaire suivante :
a. le dispositif de détection (50) est configuré pour détecter indirectement des opérations de déchargement par l'intermédiaire de l'air entrant par le canal de ventilation (40),
de préférence avec la caractéristique supplémentaire :
b. le dispositif de détection (50) dispose d'une horloge interne et est configuré pour comparer des moments d'utilisation planifiés et des opérations de déchargement effectuées et pour émettre un signal d'avertissement par l'intermédiaire d'un dispositif de signalisation (67) en cas d'absence d'opérations de déchargement aux moments d'utilisation planifiés.

6. Distributeur (10) selon l'une quelconque des revendications 2 à 5, avec au moins l'une quelconque des caractéristiques supplémentaires suivantes :
a le distributeur (10) est configuré sous forme de distributeur de flacons pressables et dispose d'un réservoir de liquide (30) compressible manuellement de l'extérieur à des fins de déchargement de liquide, et/ou
b. le réservoir de liquide (30) présente un volume maximal compris entre 5 ml et 500 ml, de préférence entre 5 ml et 50 ml, et/ou
c. le distributeur (10) est configuré sous forme de distributeur de gouttes (10) et dispose, dans la zone de l'ouverture de déchargement (22), d'une surface de formation de gouttes (23), qui est notamment délimitée sur le côté extérieur par une arête de rupture, et/ou
d. le distributeur est configuré sous forme de distributeur pharmaceutique (10) et présente un réservoir de liquide (30) rempli d'un liquide pharmaceutique, notamment d'un liquide pharmaceutique pour l'application ophtalmique.

7. Distributeur (10) selon l'une quelconque des revendications 2 à 6, avec la caractéristique supplémentaire suivante :
a. le dispositif de détection (50) est configuré sous la forme d'un module de détection externe (80) qui peut être monté sur le boîtier (20) du distributeur (10), de préférence sans outil, et peut en être retiré, de préférence sans outil,
notamment, de préférence, avec la caractéristique supplémentaire :
b. le module de détection (80) dispose d'un commutateur (69) qui peut être actionné en fixant le module de détection (80) sur le boîtier (20).

8. Distributeur (10) pour décharger un liquide selon l'une quelconque des revendications 2 à 6, avec la caractéristique supplémentaire suivante :
a. le dispositif de détection (50) est intégré dans le capuchon de protection (14).

9. Distributeur (10) pour décharger un liquide selon l'une quelconque des revendications précédentes, avec la caractéristique supplémentaire suivante :
a. le dispositif de détection (50) dispose d'un commutateur (69) qui peut être actionné par la mise en place du capuchon de protection (14) sur le boîtier (20).

10. Distributeur pour décharger un liquide selon l'une quelconque des revendications précédentes, avec la caractéristique supplémentaire suivante :
a. le dispositif de détection (50) est configuré pour déterminer le courant d'air dans le canal de ventilation (40) en réponse à une mise en place du capuchon de protection (14).
